(19) Europäisches Patentamt
European Patent Office
Office européen des brevets

(11) Veröffentlichungsnummer **0 007 093**
**B1**

# (12) EUROPÄISCHE PATENTSCHRIFT

(45) Veröffentlichungstag der Patentschrift: **20.05.81**

(51) Int. Cl.³: **C 07 D 265/30** // A01N43/84

(21) Anmeldenummer: **79102377.3**

(22) Anmeldetag: **11.07.79**

(54) **Verfahren zur Herstellung von stereoisomeren N-Aralkyl-2,6-dimethylmorpholinen.**

(30) Priorität **14.07.78 DE 2830999**

(43) Veröffentlichungstag der Anmeldung: **23.01.80 Patentblatt 80/2**

(45) Bekanntmachung des Hinweises auf die Patenterteilung: **20.05.81 Patentblatt 81/20**

(84) Benannte Vertragsstaaten
**AT BE CH DE FR GB IT LU NL SE**

(56) Entgegenhaltungen:
**DE-A-2 118 283**
**DE-A-2 656 747**
**DE-A-2 700 680**
**DE-C-1 179 947**
**HOUBEN–WEYL »Methoden der organischen Chemie«**
**Band 4, Teil 2, Seiten 276–283**

(73) Patentinhaber **BASF Aktiengesellschaft, Carl-Bosch-Strasse 38, D-6700 Ludwigshafen (DE)**

(72) Erfinder **Goetz, Norbert, Dr. Chem., Schoefferstrasse 25, D-6520 Worms (DE)**
Erfinder **Hupfer, Leopold, Dr. Chem., Waltershöhe 3, D-6701 Friedelsheim (DE)**

**EP 0 007 093 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# Verfahren zur Herstellung von stereoisomeren N-Aralkyl-2,6-dimethylmorpholinen

Die Erfindung betrifft ein Verfahren zur Herstellung von stereoisomeren N-Aralkyl-2,6-dimethylmorpholinen durch Umsetzung von Aralkanalen mit stereoisomeren 2,6-Dimethyl-morpholinen.

Es ist bekannt, daß tertiäre Amine durch Umsetzung von sekundären Aminen mit Carbonylverbindungen in Gegenwart von Wasserstoff und einem Hydrierkatalysator erhalten werden können (Houben—Weyl »Methoden der organischen Chemie«, 4/2, S. 328). In der DE-OS 2 118 283 wird die Herstellung von tertiären aliphatischen oder cycloaliphatischen Aminen beschrieben, die durch Umsetzung von aliphatischen oder cycloaliphatischen Carbonylverbindungen mit sekundären aliphatischen oder cycloaliphatischen Aminen in Gegenwart von Wasserstoff und einem Palladium-Silber-Katalysator erhalten werden können. Der Vorteil dieses Katalysators besteht darin, daß man mit ihm die reduktive Alkylierung von sekundären aliphatischen oder cycloaliphatischen Aminen in hoher Ausbeute und weitgehend frei von Nebenprodukten durchführen kann. Die bei reduktiven Alkylierungen von Aminen häufig zu beobachtende Nebenreaktion, die Hydrierung der Carbonylverbindungen unter Bildung der entsprechenden Alkohole, wird mit diesem Katalysator vermieden. Weiterhin ist es aus der DE-PS 1 179 947 bekannt, daß die Synthese N-alkylierter aromatischer Amine durch reduktive Alkylierung aromatischer Amine an einem Palladium-Silber-Katalysator unter Schonung des aromatischen Systems abläuft.

Es wurde nun gefunden, daß man stereoisomere N-Aralkyl-2,6-dimethylmorpholine der Formel I

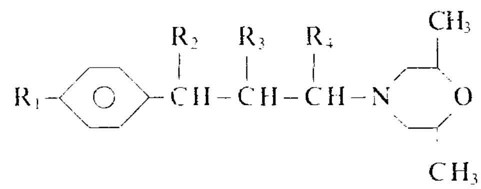

(I)

in der $R_1$ ein Wasserstoffatom, einen aliphatischen Rest mit 1 bis 10 Kohlenstoffatomen oder eine Alkoxygruppe mit 1 bis 6 Kohlenstoffatomen, $R_2$, $R_3$ und $R_4$ jeweils ein Wasserstoffatom oder einen aliphatischen Rest mit 1 bis 4 Kohlenstoffatomen bedeuten oder definierte Mischungen dieser Verbindungen durch Umsetzung von stereoisomeren 2,6-Dimethylmorpholinen der Formel II

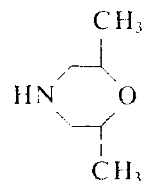

(II)

mit Verbindungen der Formel III

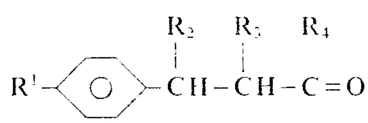

(III)

in der $R_1$, $R_2$, $R_3$ und $R_4$ die oben angegebenen Bedeutungen haben, in einfacher Weise erhält, wenn man die Umsetzung in Gegenwart von Wasserstoff und einem Hydrierkatalysator durchführt, der Palladium im Gemisch mit Silber auf einem inerten Träger enthält.

Die Umsetzung führt man vorteilhaft bei Temperaturen zwischen 100 und 190°C und Drucken zwischen 5 und 300 bar durch.

Der Vorteil des erfindungsgemäßen Verfahrens besteht darin, daß an dem verwendeten Hydrierkatalysator unter den angegebenen Bedingungen keine Isomerisierungen stattfinden, so daß man beispielsweise — ausgehend von reinem cis-2,6-Dimethylmorpholin der Formel II durch Umsetzung mit Carbonylverbindungen der Formel III — zu den sterisch einheitlichen, reinen cis-Verbindungen der Endprodukte I gelangt.

Bekannt ist, daß Isomerisierungen an Hydrierkatalysatoren durchgeführt werden können und daß bei Verwendung von hydrierend wirkenden Katalysatoren für reduktive Alkylierungen von Aminen oder für Hydrierungen mit Isomerisierungen als Nebenreaktionen zu rechnen ist (Houben—Weyl »Methoden der organischen Chemie«, 4/2, S. 276—283).

Die Aminkomponente kann nach dem erfindungsgemäßen Verfahren in stöchiometrischer Menge oder in einem bis zu 10fach molaren Überschuß angewendet werden.

Als Ausgangsstoffe entsprechend Formel III kommen beispielsweise die folgenden Carbonylverbindungen in Frage:

3-Phenyl-3-methyl-propanal, 3-Phenyl-2-methyl-propanal, 4-Phenyl-butan-2-on, 3-[4'-Methylphenyl]-2-methylpropanal, 3-[4'-Isopropylphenyl]-2-methyl-propanal, 3-[4'-Tertiärbutylphenyl]-2-methyl-propanal, 3-Phenyl-2-äthyl-propanal, 3-Phenyl-2-isopropyl-propanal, 3-[4'-Isopropylphenyl]-3-methyl-propanal, 3-[4'-Tertiärbutylphenyl]-3-methyl-propanal, 3-[4'-Tertiärbutylphenyl]-butan-4-on, 3-[4'-Methoxyphenyl]-2-methyl-propanal, 3-[4'-Isopropoxyphenyl] 2-methylpropanal.

Die Herstellung der Ausgangsstoffe III erfolgt aus den entsprechenden substituierten Benzaldehyden und aliphatischen Aldehyden bzw. Ketonen durch Aldolkondensation und anschließende partielle Hydrierung der entstandenen Kohlenstoff-Kohlenstoff-Doppelbindung (US-PS 2 976 321). Ein Teil der Verbindungen läßt sich auch durch Hydroformylierung von substituierten Styrolen herstellen.

Die Umsetzung wird vorteilhaft bei Temperaturen zwischen 100 und 190°C und einem Wasserstoffdruck zwischen 5 und 300 bar durchgeführt. Es kann ohne Lösungsmittel oder in Anwesenheit von Lösungsmitteln, die unter den Reaktionsbedingungen inert sind, gearbeitet werden.

Als Lösungsmittel kommen beispielsweise in Frage: Cyclohexan, Hexan, Heptan, Toluol, o-, m- und p-Xylol, Äthylbenzol, Di-n-butyläther, Cyclohexylmethyläther, Anisol, Methanol, Äthanol, Isopropanol, Tetrahydrofuran, Dioxan. Die Umsetzung ist sowohl kontinuierlich als auch diskontinuierlich durchführbar. Die Herstellung des verwendeten Katalysators aus Silber und Palladium auf einem inerten Träger ist aus der DE-PS 1 179 947 und der DE-OS 2 118 283 bekannt. Die katalytisch wirksamen Metalle werden zweckmäßig in einem solchen Verhältnis verwendet, daß, bezogen auf Silber, 1 bis 30, insbesondere 3 bis 15 Gewichtsprozent Palladium vorhanden sind. Manchmal läßt sich der Katalysator noch durch einen Zusatz von bis zu 50, insbesondere bis zu 25 Gewichtsprozent, bezogen auf Silber, an Mangan oder Vanadiumoxyd in seiner Wirksamkeit verbessern. Als Träger können beispielsweise Kieselsäure oder hocherhitztes und wieder abgekühltes Aluminiumoxyd verwendet werden. Das Gewichtsverhältnis Trägerstoff zu Metall beträgt beispielsweise 93 zu 7 Gewichtsteile. Man verwendet den Katalysator beispielsweise in Strängen von 5 mm Durchmesser und 10 mm Länge.

Die nach dem Verfahren der Erfindung hergestellten Verbindungen finden als Wirkstoffe in bekannten Pflanzenschutzmitteln Verwendung (DE-OS 2 656 747).

Die in den folgenden Beispielen genannten Teile bedeuten Gewichtsteile; Raumteile verhalten sich zu ihnen wie Liter zu Kilogramm.

## Beispiel 1

In ein zylindrisches Reaktionsrohr mit einem Volumen von 500 Raumteilen wird ein Katalysator mit der Zusammensetzung von 0,36 Gew.-% Pd, 4,8 Gew.-% Ag und 1,05 Gew.-% Mn auf $SiO_2$ als Träger eingefüllt und auf 150°C erhitzt. Über dieses Katalysatorbett werden stündlich 120 Teile einer Mischung geführt, die aus 204 Teilen 3-[p-Tertiärbutylphenyl]-2-methyl-propanal und 115 Teilen cis-2,6-Dimethylmorpholin besteht. Gleichzeitig werden 10 000 Raumteile Wasserstoff bei einem Druck von 200 bar durch das Reaktionsrohr im Gleichstrom hindurchgeleitet. Das aus dem Reaktionsrohr herausfließende Reaktionsprodukt wird unter Druck abgekühlt und dann entspannt. Es fallen hierbei stündlich ca. 120 Teile Rohprodukt an, die destillativ gereinigt werden. Die Destillation von 120 Teilen Rohprodukt führt zu 107 Teilen N-[3'-Tertiärbutylphenyl)-2'-methyl-propyl]-cis-2,6-dimethylmorpholin, $Kp_{18} = 206°$ C. Das entspricht einer Ausbeute von 94% der Theorie.

## Beispiel 2

In der gleichen Apparatur und unter den gleichen Bedingungen im gleichen Mengenverhältnis — wie in Beispiel 1 beschrieben — wird 3-[p-Tertiärbutylphenyl]-2-methyl-propanal mit einem Gemisch aus 75 Gew.-% cis-2,6-Dimethylmorpholin und 25 Gew.-% trans-2,6-Dimethylmorpholin umgesetzt. Hierbei wird als Endprodukt ein Gemisch aus 75 Gew.-% N-[3'-(p-Tertiärbutylphenyl)-2'-methyl-propyl]-cis-2,6-dimethylmorpholin und 25 Gew.-% N-[3'-(p-Tertiärbutylphenyl)-2'-methyl-propyl]-trans-2,6-dimethylmorpholin, $Kp_{18} = 206-210°$ C, erhalten. Die Ausbeute beträgt 94% d. Th.

## Beispiel 3

Ein Schüttelautoklav mit einem Volumen von 250 Raumteilen wird mit 50 Teilen 3-(p-Isopropylphenyl)-3-methyl-propanal, 100 Teilen trans-2,6-Dimethylmorpholin und 10 Teilen des in Beispiel 1 genannten Katalysators gefüllt. Anschließend wird der Autoklav auf 140°C erhitzt und Wasserstoff bis

zum Erreichen eines Druckes von 200 bar aufgepreßt. Sobald die Wasserstoffaufnahme beendet und ein konstanter Druck erreicht ist, wird abgekühlt.

Danach wird der Katalysator vom Reaktionsprodukt durch Filtration abgetrennt und das Filtrat destillativ gereinigt. Man erhält 69 Teile N-[3'-(p-Isopropylphenyl)-3'-methyl-propyl]-trans-2,6-dimethylmorpholin, $Kp_{0,01} = 132°$ C. Die Ausbeute beträgt 91% d. Th.

Beispiel 4

Der unter Beispiel 3 angegebene Schüttelautoklav wird mit 50 Raumteilen 3-Phenyl-3-methyl-propanal, 100 Teilen cis-2,6-Dimethylmorpholin und 10 Teilen des in Beispiel 1 genannten Katalysators gefüllt. Anschließend wird der Autoklav auf 160°C erhitzt und Wasserstoff bis zum Erreichen eines Druckes von 200 bar aufgepreßt. Sobald die Wasserstoffaufnahme beendet und ein konstanter Druck erreicht ist, wird abgekühlt. Danach wird der Katalysator vom Reaktionsprodukt durch Filtration abgetrennt und das Filtrat destillativ gereinigt. Man erhält 77,5 Teile N-(3'-Phenyl-3'-methyl-propyl)-cis-2,6-dimethylmorpholin, $Kp_{0,1} = 103°$ C. Die Ausbeute beträgt 93% d. Th.

Beispiel 5

Man verfährt analog Beispiel 4, verwendet aber als Ausgangsstoffe 3-(p-Methoxyphenyl)-2-methylpropanal und cis-2,6-Dimethylmorpholin. Die destillative Aufarbeitung des Reaktionsproduktes liefert hier N-[3'-(p-Methoxyphenyl)-2'-methyl-propyl]-cis-2,6-dimethylmorpholin, $Kp_{0,1} = 128°$ C. Die Ausbeute beträgt 92% d. Th.

Beispiel 6

Man verfährt analog Beispiel 4, verwendet aber als Ausgangsstoffe 4-(p-Tertiärbutylphenyl)-butan-2-on als Carbonylkomponente und als Aminkomponente ein Gemisch aus 75 Gew.-% cis- und 25 Gew.-% trans-2,6-Dimethylmorpholin. Die destillative Aufarbeitung führt zu einem Gemisch aus 75 Gew.-% N-[4'-(p-Tertiärbutylphenyl)-but-2'-yl]-cis-2,6-dimethylmorpholin und 25 Gew.-% N-[4'-(p-Tertiärbutylphenyl)-but-2'-yl]-trans-2,6-dimethylmorpholin, $Kp_{0,01} = 143°$ C. Die Ausbeute beträgt 86% d. Th.

Beispiel 7

Man verfährt analog Beispiel 4, verwendet aber einen Katalysator mit der Zusammensetzung von 0,3 Gew.-% Pd und 4,5% Ag auf $SiO_2$, als Carbonylkomponente 3-[p-Tertiärbutylphenyl]-2-methyl-propanal und als Aminkomponente cis-2,6-Dimethylmorpholin.

Die destillative Aufarbeitung des Reaktionsproduktes führt zu N-[3'-(p-Tertiärbutylphenyl)-2'-methyl-propyl]-cis-2,6-dimethylmorpholin, mit einer Ausbeute von 92% d. Th.

**Patentansprüche**

1. Verfahren zur Herstellung von stereoisomeren N-Aralkyl-2,6-dimethylmorpholinen der Formel I

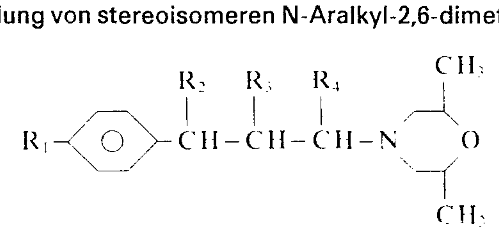

(I)

wobei $R_1$ ein Wasserstoffatom, einen aliphatischen Rest mit 1 bis 10 Kohlenstoffatomen oder eine Alkoxygruppe mit 1 bis 6 Kohlenstoffatomen, $R_2$, $R_3$ und $R_4$ jeweils ein Wasserstoffatom oder einen aliphatischen Rest mit 1 bis 4 Kohlenstoffatomen bedeuten oder definierten Mischungen dieser Verbindungen durch Umsetzung von stereoisomeren 2,6-Dimorpholinen der Formel II

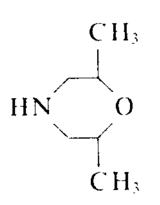

(II)

oder definierten Mischungen dieser Verbindungen mit Verbindungen der Formel III

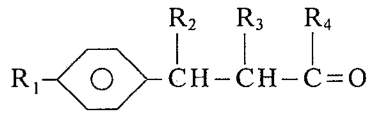

(III)

wobei $R_1$, $R_2$, $R_3$ und $R_4$ die oben angegebenen Bedeutungen haben, dadurch gekennzeichnet, daß man die Umsetzung in Gegenwart von Wasserstoff und einem Hydrierkatalysator durchführt, der Palladium im Gemisch mit Silber auf einem inerten Träger enthält.

2. Verfahren nach Anspruch 1, dadurch gekennzeichnet, daß ein Katalysator verwendet wird, der neben Palladium und Silber zusätzlich noch Mangan oder Vanadiumoxyd enthält.

3. Verfahren nach Anspruch 1, dadurch gekennzeichnet, daß man die Umsetzung in einem Temperaturbereich von 100 bis 190° C und einem Druckbereich von 5 bis 300 bar durchführt.

**Claims**

1. A process for the preparation of a stereoisomeric N-aralkyl-2,6-dimethylmorpholine of the formula I

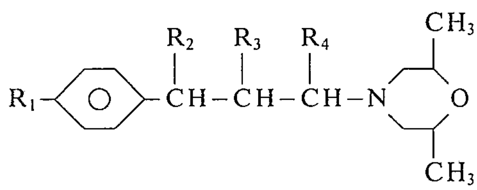

(I)

where $R_1$ is hydrogen, an aliphatic radical of 1 to 10 carbon atoms or alkoxy of 1 to 6 carbon atoms and $R_2$, $R_3$ and $R_4$ each denote hydrogen or an aliphatic radical of 1 to 4 carbon atoms, or of a defined mixture of such compounds, by reacting a stereoisomeric 2,6-dimorpholine of the formula II

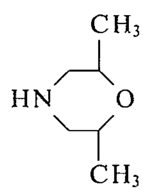

(II)

or a defined mixture of such compounds with a compound of the formula III

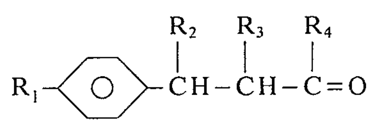

(III)

where $R_1$, $R_2$, $R_3$ and $R_4$ have the above meanings, characterized in that the reaction is carried out in the presence of hydrogen and of a hydrogenation catalyst which contains palladium mixed with silver on an inert carrier.

2. A process as claimed in claim 1, characterized in that a catalyst is used which contains manganese or vanadium oxide in addition to palladium and silver.

3. A process as claimed in claim 1, characterized in that the reaction is carried out at a temperature of from 100° to 190° C and a pressure of from 5 to 300 bars.

**Revendications**

1. Procédé de préparation de N-aralkyl-2,6-diméthylmorpholines de formule I

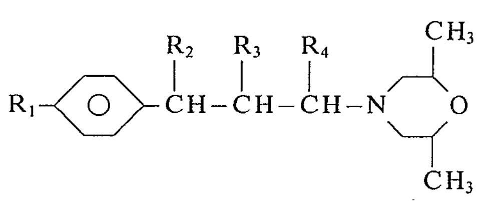

(I)

dans laquelle $R_1$ représente un atome d'hydrogène, un reste aliphatique en $C_1$ à $C_{10}$ ou un groupe alcoxy

en $C_1$ à $C_6$, $R_2$, $R_3$ et $R_4$ chacun un atome d'hydrogène ou un reste aliphatique en $C_1$ à $C_4$, ou des mélanges définis de ces composés, par réaction de 2,6-dimorpholines stéréoisomères de formule II

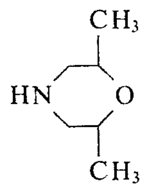

(II)

ou des mélanges définis de ces composés avec des composés de formule III

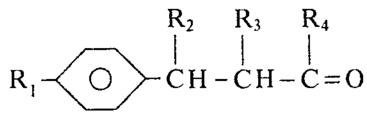

(III)

$R_1$, $R_2$, $R_3$ et $R_4$ ayant les significations indiquées cidessus, caractérisé par le fait qu'on effectue la réaction en présence d'hydrogène et d'un catalyseur d'hydrogénation qui contient du palladium en mélange avec de l'argent sur un support inerte.

2. Procédé selon la revendication 1, caractérisé par le fait qu'on utilise un catalyseur qui, outre le palladium et l'argent, contient encore du manganèse ou de l'oxyde de vanadium.

3. Procédé selon la revendication 1, caractérisé par le fait qu'on effectue la réaction dans une zone de température de 100 à 190° C et une zone de pression de 5 à 300 bars.